# EUROPEAN PATENT APPLICATION

(11) **EP 2 077 131 A1**
(43) Date of publication of application: **08.07.2009**
(21) Application number: 07816620.4
(22) Date of filing: 22.10.2007
(51) Int. Cl.: A61M 5/158, A61M 5/32, A61M 25/06

(54) **SAFETY TRANSFUSION CATHETER**

(30) Priority: 23.10.2006 CN 200610117448; 23.10.2006 CN 200620047022 U; 23.10.2006 CN 200620047024 U
(71) Applicant: Sun Medical-Scientific (Shanghai) CO., LTD., Shanghai 201318 (CN)
(72) Inventor: SHEN, Luyi, Shanghai 201201 (CN)
(74) Representative: Brown, David Leslie
(86) International application number: PCT/CN2007/003007
(87) International publication number: WO 2008/049331

(57) **Abstract**

A safety transfusion catheter comprises a flexible retention tube (2) fitted over a metal guiding needle (1). The flexible retention tube (2) is provided with a needle handle (22;22') connected with it. The angle between the end (3) of the flexible retention tube (2) and its longitudinal axis or longitudinal tube wall is less than 90°. The cross section of the needle handle (22;22') has an outer contour line of a closed curve formed with a curve (5;25) intersecting a beeline (6;26). A further safety transfusion catheter comprises a flexible retention tube (39) fitted over a metal guiding needle (32), a transfusion connecting tube (38) in fluid communication with flexible retention tube (39) and a rubber casing cap (42) at the end of the flexible retention tube (39). The back end of the metal guiding needle (32) is in telescoping engagement with a hollow sleeve (31). The transfusion connecting tube (38) and flexible retention tube (39) form a T-shaped or an oblique Y-shaped structure. A safety protect sheath (514) shorter than the metal guiding needle (32) is provided at the end of the hollow sleeve (31). A spring (533) is provided between the end hub (34) of the metal guiding needle (32) and the front end of the hollow sleeve (31). A seesaw typed stop block (36) is provided in the hollow sleeve (31). A reset button (37) is provided on the portion of the hollow sleeve (31) having the seesaw typed stop block (36).

## Description

### Related Applications

The present application is related to Chinese Provisional Patent Application serial no. 200610117448.7, 200620047022.4, 200620047024.3, all of them filed on October 23, 2006, which is incorporated herein by reference and to which priority is claimed pursuant to

### Background of the Invention

### 1. Field of the Invention

The invention relates to the field of medical device that is used for infusion liquid into the vessel of human body.

### 2. Description of the Prior Art

Intravenous infusion currently remains the major procedure for clinical care and treatment, particularly, in the intensive care unit, emergency room, etc., immediately establishing the intravenous transfusion is often the key elements of life-saving. Rapidly establishing the intravenous transfusion depends on both a health care professional's first-stick success technique, and the patient's vein thickness and its position. The traditional intravenous infusion technique is using a metal needle to penetrate the vessel for infusing liquid medicine. After infusion, the needle has to be pulled out. In most cases, repeated infusion procedure is needed for treatment. Therefore, patients have to suffer from needle stick for each infusion. In patients with chronic diseases, longtime infusion is often needed. In these patients, steel needle has to be remained in the patients' vessel for a long time. Sometime the steel needle tip penetrates the vessel wall while the limb is moved. This failure of the infusion can cause bleeding and fluid extravasation that can cause pain and surrounding tissue edema and damage. Thus, the needle must be pulled out and another vessel penetration is not avoidable,

Current technology has used the soft plastic catheter for intravenous infusion (Chinese Patent CN2163653Y, 05/041994). After a soft plastic catheter as a sleeve on the steel needle be used for penetrating the vessel wall, the steel needle will be pulled out, then the catheter will be remained in the vessel for infusion. The catheter will then be connected to the intravenous infusion set, for i.v. infusion. Thus, the soft plastic catheter will not injured and penetrate the vessel wall during infusion as that seen in needle set, the repeated needle stick is avoid.

However, all of infusion catheters have the blunt-cutting edge on its tip. The flat edge is 90°C to the long axis catheter wall (Chinese patent CN1468637 figure 3). The catheter tip is relatively soft and blunt. It is not so easy to be inserted into the vessel wall as the steel needle tip, since there is certain resistance when it penetrating through the vessel wall. Sometimes, the edge of the tip will be dilated as "bell" shape, or tear, due to the extra force is used for insertion, the catheter can not be inserted into the vessel, then the catheter must be changed and reinserted..

Additionally, the design of the handle of the catheter-needle currently available is round shape, it is not easy to be fixed on the body surface. This is another disadvantage of the current design.

On another hand, recent advance in the design of infusion catheter has been focused on effectively safeguard healthcare workers from accidental needlestick injuries. In a Chinese patent CN1135122C, published in 01/21/2004 has shown the design of a "multi-purpose safety intravenous infusion catheter needle" that consist with a trifurcate connector, an infusion soft needle, a steel needle, and an infusion catheter. The first and second connector on the trifurcate connector is the one the same axial and toward to the needle tip. There is a rubber cap on the end of the second connector. The soft needle catheter is on the first connector. The steel needle body slidably fitted in a sleeve and axially passed in the soft needle. The sleeve is connected with the second connecting section of the trifurcate connector. An infusion catheter is connected with a third connecting section of the trifurcate connector. After the soft needle catheter inserted into the patient's vein with the steel needle, the steel needle will be pulled out from the end of the sleeve, the needle will be kept in the sleeve, this improvement can avoid the needle exposed, which can caused accidental needle-stick injury to the others, then cause infection.

However, this design still has some short-comes:
1) The steel needle has to be pulled out from the soft needle catheter manually, this distance is not so short, so it is not so convenient for the operator;
2) The trifurcate is not so easy to handle. Specially in patients with obesity, excessive bleeding, dehydrate, etc., the vein is hard to find, it is difficult to find the vein and insert the needle catheter quickly and accurately for the healthcare professional. Thus, it is not only waist the time, and also can cause the unnecessary suffering for the patients.
3) The outside contours of the cross section profile of the trifurcate's soft needle catheter is round shaped, it is difficult to be fixe on the patients' body surface, it is also easy to be rotated or moved after it be fixed during the infusion.

### Brief Description of the Content of the Invention

The purse of this invention is to find the solution for technical problems in the design of a safety infusion needle catheter for infuse the liquid into the vessel, which can fulfill the requirement for the long-time indwelling the infusion catheter in the vein, and also can be used for more than one time penetrating the vessel wall, easy for handling, easy for fixing, thus, the vessel injury can be minimized, the infusion process can be carried on quickly, and easily, therefore the patient's unnecessary suffering will be reduced.

The technical strategy of the present invention is: to provide safety infusion catheter comprise a flexible retention tube fitted over a metal guide needle with a wedge-shaped needle tip. The flexible retention tube is provided with a needle handle connected with it. The angle between the end of the flexible retention tube and its longitudinal axis or longitudinal tube wall is less than 90°. The cross section of the needle handle has an outer contour line of a closed curve formed with a curve intersecting a beeline.

Specifically, the cutting line of the end of the flexible retention tube is parallel to the opening of the metal guide needle. There is a chamfer structure on the opening end of the wall of the flexible retention tube. The surface of the edge of the opening of the flexible retention tube is polished. There is a round-smooth transition made in the connection between the edge of the opening and the out wall of the flexible retention tube. The curve described above is a circular arc curve, elliptical arc curve, droplet-type curve or their combinations.

Furthermore, the outer contour line of the cross section of the needle handle described above is semicircular, semielliptical, semi drop-shaped, approximate semicircular, approximate semielliptical, approximate semi drop-shaped, or their combinations. The needle handle described above is a two pieces-hinge structure that can be buckled together. There are hasp buckles on the two pieces-hinge structure, respectively. On the inner face-to-face side of two pieces-hinge structure there is a furrow that allows flexible retention tube to be lay between two pieces-hinge structures. On the surface of the needle handle there are embossing non-skid tread.

The present invention provides a total-safety transfusion catheter comprises a flexible retention tube fitted over a metal guiding needle, a fluid transfusion connecting tube in fluid communication with flexible retention tube and a rubber or silicon casing plug shaped with thinner at the center area at the distal end of the flexible retention tube. The back end of the metal guiding needle is in telescoping engagement with a hollow sleeve. The transfusion connecting tube and flexible retention tube form a T-shaped or an oblique Y-shaped structure. A safety protect sheath shorter than the metal guiding needle is provided at the end of the hollow sleeve. A spring is provided between the end hub of the metal guiding needle and the front end of the hollow sleeve. A seesaw typed stop block is provided in the hollow sleeve. A reset button is provided on the portion of the hollow sleeve having the seesaw typed stop block.

Specifically, the metal guiding needle is slidably fitting in the hollow protection sleeve. The spring is put around the metal guiding needle. A seesaw type stop blocker is fitted at the end of the metal guiding needle that allows the needle can be completely out of the sleeve into to the flexible catheter tube. An additional locking click board is fitted on the seesaw type stop block to keep the metal needle inserted through the rubber (or silicon) plug into the flexible catheter tube while it in the operating position even when the tip of the metal guiding needle be pulled out from the rubber (or silicon) plug manually. Sliding the locking click board to release the seesaw type stop block, the tip of the metal guiding needle can be completely retracted into the safety sleeve.

Furthermore, in the conjunction of the infusion tube connection and the flexible infusion tube described above adding a wing-shaped or a butterfly-shaped adjunct handle. One side of the handle is flat, on the surface of at lest one side of the handle has embossing non-skid tread. The handle is a two pieces-hinge structure buckled together, on the conjunction of the infusion tube connection and the flexible infusion tube.

### Brief Description of the Drawings

Fig. 1 shows the schematic drawing of the end of the flexible infusion catheter of the present invention.
Fig. 2 is the diagram of the implementation of case of Fig 1.
Fig. 3 is the diagram of another implementation of case of Fig 1.
Fig. 4 is the structure diagram of the needle handle of the present invention
Fig. 5 is the A-A' sectional view of Fig. 4.
Fig. 6 is another structure diagram of the needle handle of the present invention
Fig. 7 C section view of Fig. 6.
Fig. 8 is the structure diagram of the metal guiding needle component of the present invention.
Fig. 9 is the structure diagram of the auxiliary needle handle.
Fig. 10 is the A-A' sectional view of Fig. 9.
Fig. 11 is structure diagram of the second embodiment of the metal guiding needle component of the present invention.
Fig. 12 is s perspective assembled view of the plug-in connection between the infusion catheter and the conjunction of the flexible infusion catheter, needle and the distal end of the infusion catheter.

### Detailed Description of the Preferred Embodiments

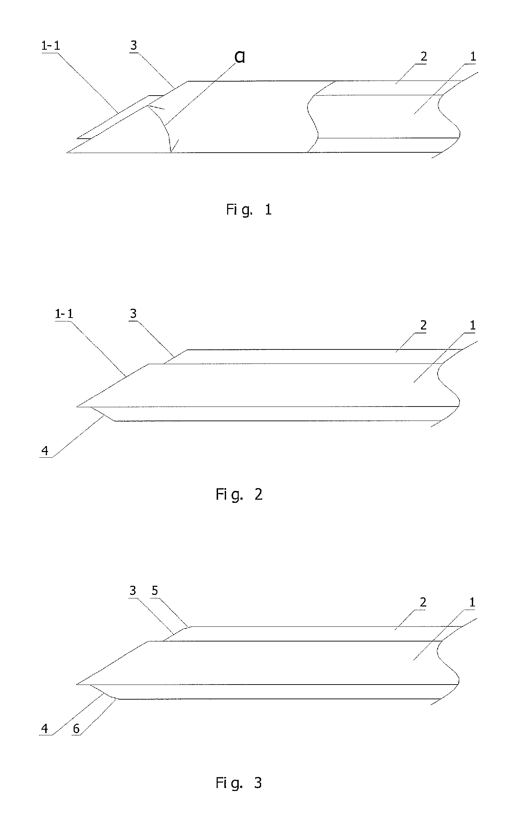

In Fig. 1, the present invention consists with a metal guide needle 1 with the wedge-shaped needle tipl-1 fitted in the flexible infusion needle catheter 2. The key element is the angel between the edge of the flexible infusion needle catheter tube 3 and the longitudinal axis of catheter tube is less than 90°.

Furthermore, the edge of the flexible infusion needle catheter tube 3 is parallel with the edge of the wedge-shaped metal needle tip 1-1.

The cross section of the edge of the flexible infusion needle catheter tube 3 is made with non-planar, wedge-shaped cutting that will reduce the resistance of flexible infusion catheter while penetrating the vessel and during further advance into the vessel after penetration.

Referring to Fig 2, to further reduce the resistance of flexible infusion catheter during penetration and insertion, a chamfer 4 was made on the edge of flexible infusion catheter tip 3 wall.

Remaining is the same as described above.

In Fig. 3, there is a round-smooth transition made in the connection between the edge of the opening and the out wall of the flexible retention tube.

The round-smooth transition structure could also reduce the resistance of flexible infusion catheter during penetration and insertion, prolonged the using-life, improve the first-stick success, and it will also minimize the vessel wall injury during penetration and insertion.

Remaining is the same as described above.

In considering simplifying the processing technology, as a simple structure, a non-planner arc structure can be used on the edge of the flexible infusion catheter tip 3.

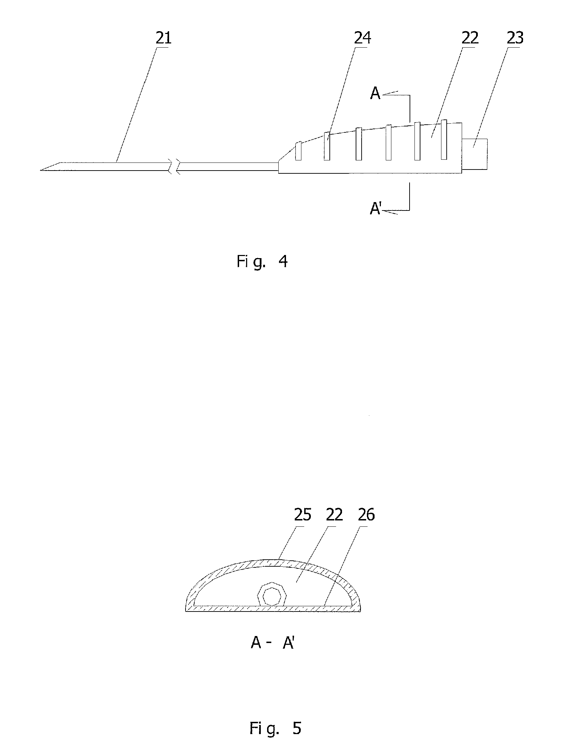

In Fig. 4, the present invention consist with needle 21 and the needle handle 22 that is connected with the needle. On the outside surface of the needle handle, there are embossing non-skid tread 24. In the distal end of the needle handle, there is a connection for the infusion catheter 23.

In Fig. 5, the outside contour line of the cross section of the needle handle 22 is a closed curve formed with a curve intersecting a beeline. This curve could be a circular arc curve, elliptical arc curve, droplet-type curve or their combinations.

Additionally, the outer contour line of the cross section of the needle handle could be the semicircular, semielliptical, semi drop-shaped, approximate semicircular, approximate semielliptical, approximate semi drop-shaped, or their combinations.

As it shown in the Fig. 5, the outside contour line of the cross section of the needle handle could be a closed curve formed with a circular arc line 5 intersecting a beeline 6.

Since one side of the needle handle is plane, this needle handle is easy to be fixed on the patient's body surface.

Remaining is the same as described above in Fig. 4.

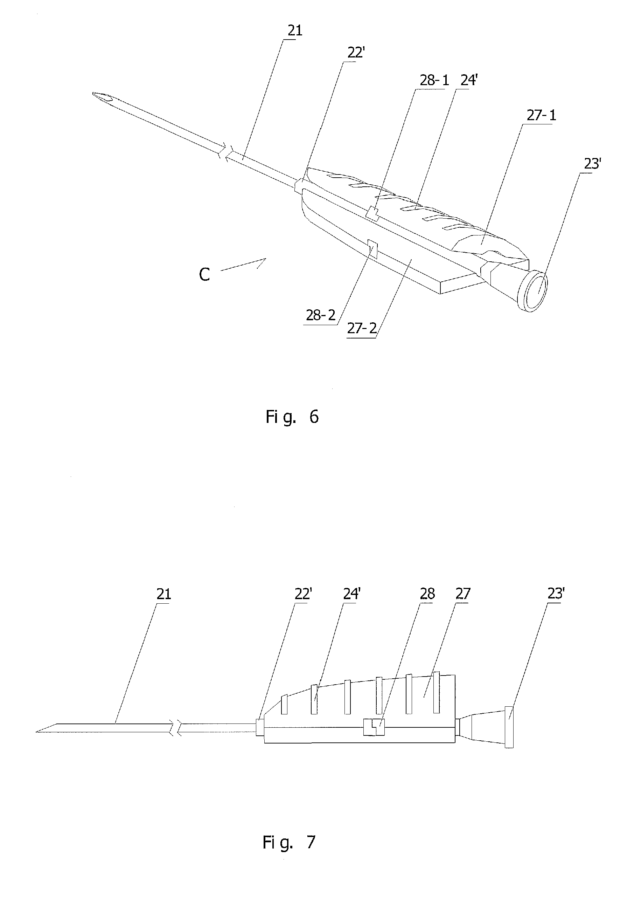

In Fig. 6, in the present invention, the needle handle 22' described above could also be used as an accessory of the transfusion needle catheter. This handle has a two-pieces-hinge structure that can be buckled together. There are two hasp buckles 28-1, 28-2 on the two pieces-hinge structure 27-1, 27-2, respectively. On the inner face-to-face side of two pieces-hinge structure there is a furrow that allows flexible retention tube to be lay between two pieces-hinge structures. The body of the needle handle is lay in the middle of two pieces-hinge structure of the needle handle.

The outer contour line of the cross section of the needle handle is similar as that shown in Fig. 5.

On the outside surface of the needle handle, there are embossing non-skid tread as well.

In Fig. 7, since this needle handle accessory is two-pieces-hinge structure that can be buckled up, so as shown in Fig. 6 the commercially available needle handle body 22'could be placed into the middle of the opened two-pieces-hinge structure 27-1, 27-2 if it has the outside shape matched. When close two-pieces-hinge structure, it will be the close-state needle handle accessory 27. When both hasp buckle are closed 28 (the open-state is shown in Fig. 6 28-1, 28-2), the two-pieces-hinge structure accessory and the needle handle 22' inside, as a whole which has the same outside structure as described above.

Remaining is the same as described above in Fig. 6.

Referring to Fig, 8, in the front-end of the sleeve cap 31 there is a safety protection sleeve 33 it is shorter than the metal guiding needle 32. A spring 35 is placed between the back-end of the metal guiding needle block 34 and the front-end of the hollow sleeve. The back end of the metal guiding needle 32 is in telescoping engagement with a hollow sleeve cap 31. A spring is provided between the end hub 34 of the metal guiding needle 32 and the front end of the hollow sleeve cap 31. A seesaw typed stop block 36 is provided in the hollow sleeve 31. A reset button 37 is provided on the portion of the hollow sleeve cap 31 having the seesaw typed stop block 36.

The metal guiding needle is slidably set in the protecting sleeve 33 and the hollow sleeve cap 31. The spring is provided on the metal guiding needle. A seesaw type stop block is provided behind the back end while the metal guiding needle is completely out of the hollow sleeve cap. An additional locking click board is fitted on the seesaw type stop block to keep the metal needle inserted through the rubber (or silicon) plug into the flexible catheter tube while it in the operating position even when the tip of the metal guiding needle be pulled out from the rubber (or silicon) plug manually. Sliding the locking click board to unlock the reset button to be pressed to release the seesaw type stop block, and let it disconnected with the back end of the needle block, the tip of the metal guiding needle can be completely retracted into the safety sleeve. This design is able to achieve the goal for single-hand handle, and quick and easy operation".

Since the protection sleeve is provided, the metal guiding needle only needs travel from the front end of the flexible infusion catheter to the front end of the safety protection sleeve, and achieve the goal of the safety, although the moving distance for the metal guiding needle is greatly decreased, the hollow sleeve cap is much shorter and thinner.

Providing the protection sleeve reduced the length of the safety hollow sleeve, and reduces the required force of spring while retracting the metal guiding needle into the safety hollow sleeve. On another hand, this design also increased its contact area with the rubber or silicon plug that prevents the metal guiding needle cutting the plug, reduces the chance for excluding open or enlarge the needle hole on the plug, resulting fluid leaking from the needle hole, keeps the sealing effect of the plug. This design can also reduce the required thickness of the rubber or silicon plug, thus further reduces the required force for the spring, increase the feasibility.

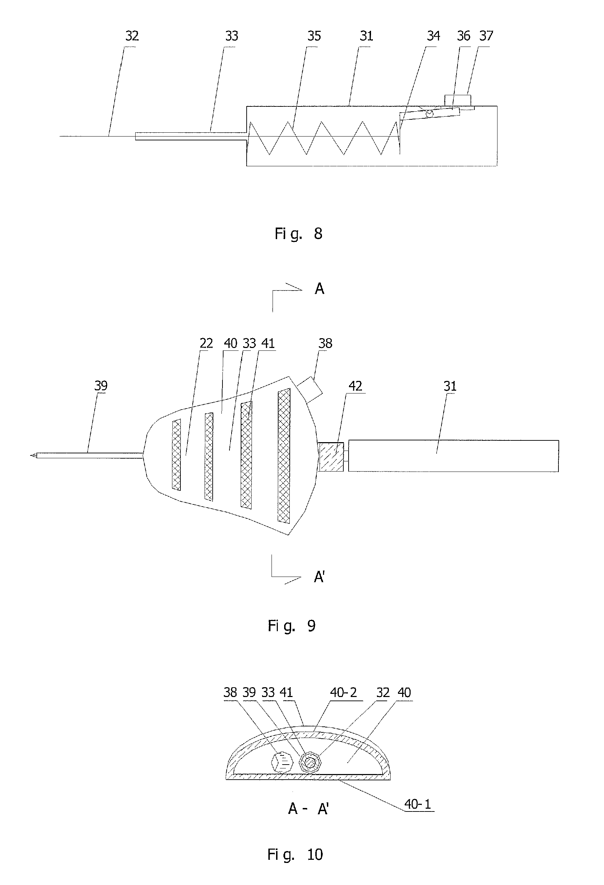

As shown in Fig. 9, the in the conjunction of infusion catheter 38 and the flexible transfusion needle catheter 39, there is a wing-shaped or a butterfly-shaped adjunct handle 40. Embossing non-skid 41 treads was provided on the surface of at least one side of the adjunct handle body.

Furthermore, the adjunct handle is a two-pieces-hinge structure that can be buckled up, and can be provided on the conjunction of the infusion tube connection and the flexible infusion needle catheter. Since this handle structure is well understand by the technical professional in this field, so it the figure is not shown.

In Fig. 10, in the side view of the adjunct needle handle 40, one side is flat side 40-1, embossing non-skid treads 41 are provided on the outside surface of another side needle handle. Providing the embossing non-skid treads on the outside surface of the needle handle made it easier to handle and operating.

As a model and expansion, a design for the detachable infusion needle catheter is show as following:

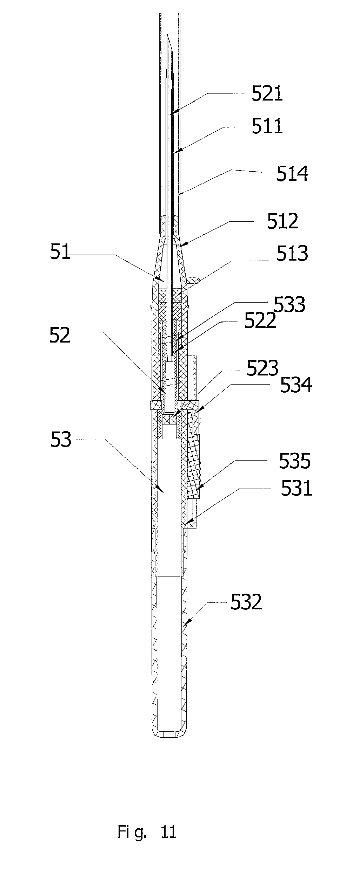

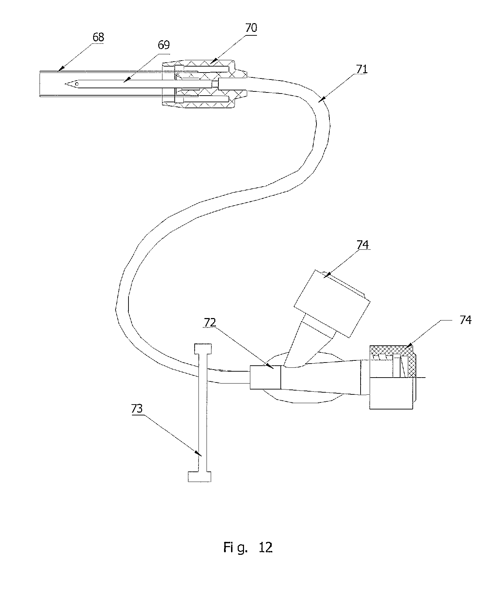

Providing a back block on the flexible infusion needle catheter, on the back-end of the back block inserts a seepage-preventing rubber or silicon plug. The set of the metal guiding needle with safety protective sleeve and its components are provided in the back block of the flexible needle catheter. A needle can be inserted into the seepage-preventing rubber or silicon plug is provided in the front end of the infusion catheter. First inserting the flexible infusion needle catheter with the metal guiding needle into the vessel, after extruding the metal guiding needle, inserts the needle on the infusion tube into the back end of the back block of the flexible infusion catheter, for fluid infusion. This can also achieve the goal and effect of this invention. The schematic illustrations of this application have been shown in Fig. 11 and 12.

In this embodiment of the invention, the illustrations in Fig. 1-10 are also applicable, for making the figures concise, they have not been shown, but this should not be used for limiting the present invention.

Fig. 11 combined with Fig. 8 illustrates the flexible infusion catheter needle, the block of flexible tube, rubber or silicon plug, and protective sleeve, The blood seepage preventing flexible needle catheter 51 in consist with flexible infusion needle catheter 551, flexible tube block 512, rubber or silicon plug 513, and protective sleeve 514.

In the front end of flexible infusion catheter provides a conical narrow part, its back end is connected with the opening of the small tube in the front end of the flexible catheter block The inner opening of the flexible catheter block connected with the inner opening of the flexible infusion needle catheter tube through the conical hole. The rubber or silicon plug is fixed in the middle of the cylindrical hole.

The metal guiding needle components 53 is consist with a protective sleeve 511, safety hollow sleeve cap 532, spring 533, seesaw typed stop block button 533, rest-locking button 535. The metal guiding needle protrudes out of the front end of the protective sleeve. A horizontal slot and the vertical chute slot are provided on the front part of the protective sleeve. The positioning plate of the seesaw typed stop block button is inserted into the slot, and embedded into the slot of the needle block, thus, the metal guiding needle is fixed in front end of the protective sleeve, and the spring is compressed before using. The reset-lock key is fitted in the chute slot, the locking plate is embedded in the bottom of the seesaw typed stop block button to prevent it be the accidently pressed before the flexible infusion needle catheter is successfully inserted into the vessel. The safety hollow sleeve cap is connected with the protective sleeve as a whole by a flip block.

When opening the reset-locking key and pressing the seesaw typed stop block button, the whole body of the metal guiding needle is retracted from the flexible infusion needle catheter and completely back into the protective sleeve and the safety hollow sleeve cap.

Remaining are the same as that in Fig. 8.

While using the design show in Fig. 11, in order to make connection between the blood seepage preventing flexible infusion needle catheter and the infusion tube, the plug-in type connection tube could be used as shown in Fig. 12, that consist with flexible infusion needle catheter tube 69, needle tube block 70, catheter 71, tube clip 73, Y shaped connecter or 3-way stopcock 72 and block cap 74.

There is an oblique cone shaped needle tip in the front end of the insertion needle, its back end connected with the front end of the needle block. The back end of the needle block is connected with the front end of the infusion catheter. Another end of the catheter connected with the Y shaped connector body. Other two end of the connecter body provide cone Ruhr lock connecter. The block cap can be used for blocking the flow before using or stop using. A 3-way stopcock can be used as an alternative setting. One end of the Y connector or 3-way stopcock can connected with the infusion set. A tube clip is provided for additional control of the flow for convenience. The over long protective needle sleeve covers the needle that is able to fit on the outside of the connector of the flexible catheter blocker.

While using the designed plug-in type infusion tube connector shown in Fig. 12 with the blood seepage preventing flexible infusion needle catheter shown in Fig. 11, first, the blood seepage preventing flexible infusion needle catheter with the metal guiding needle components are used for making the penetration into the vessel, then extrude the metal guiding needle and take out the needle components. After inserting the plug-in connector with infusion tube into the protective sleeve and the rubber or silicon plug on the blood seepage preventing flexible infusion needle catheter, then infusion can be initiated. Alternatively, the plug-in type infusion tube connector system shown in Fig. 12 can also be connected to the infusion connector through direct conjunction on the T or Y shaped safety flexible infusion needle catheter system shown in Fig. 10, and 11.

Many alterations and modifications may be made by those having ordinary skill in the art without departing from the spirit and scope of the invention. Therefore, it must be understood that the illustrated embodiment has been set forth only for the purposes of example and that it should not be taken as limiting the invention as defined by the following claims. For example, notwithstanding the fact that the elements of a claim are set forth below in a certain combination, it must be expressly understood that the invention includes other combinations of fewer, more or different elements, which are disclosed in above even when not initially claimed in such combinations.

The words used in this specification to describe the invention and its various embodiments are to be understood not only in the sense of their commonly defined meanings, but to include by special definition in this specification structure, material or acts beyond the scope of the commonly defined meanings. Thus if an element can be understood in the context of this specification as including more than one meaning, then its use in a claim must be understood as being generic to all possible meanings supported by the specification and by the word itself.

The definitions of the words or elements of the following claims are, therefore, defined in this specification to include not only the combination of elements which are literally set forth, but all equivalent structure, material or acts for performing substantially the same function in substantially the same way to obtain substantially the same result. In this sense it is therefore contemplated that an equivalent substitution of two or more elements may be made for any one of the elements in the claims below or that a single element may be substituted for two or more elements in a claim. Although elements may be described above as acting in certain combinations and even intially claimed as such, it is to be expressly understood that one or more elements from a claimed combination can in some cases be excised from the combination and that the claimed combination may be directed to a subcombination or variation of a subcombination.

Insubstantial changes from the claimed subject matter as viewed by a person with ordinary skill in the art, now known or later devised, are expressly contemplated as being equivalently within the scope of the claims. Therefore, obvious substitutions now or later known to one with ordinary skill in the art are defined to be within the scope of the defined elements.

The claims are thus to be understood to include what is specifically illustrated and described above, what is conceptionally equivalent, what can be obviously substituted and also what essentially incorporates the essential idea of the invention.

### Application:

The present invention has following advantages: the flexible retention tube is provided with a non-planar, wedge-shaped cutting edge that is parallel with the metal guiding needle cutting edge, and the less than 90° chamfer and polishing is made on the cutting edge of the tube wall. These improvements will reduce the resistance of the flexible infusion catheter during penetrating and advancing in the vessel, reduces the vessel injury and greatly improve the chance of first-stick success. One side of the needle handle is planner, that will helpful for fixing it on the patient's body surface. Providing the seesaw type stop block, reset-locking button, and the design for shorter spring moving distance, allows the metal needle fast retracting into the safety sleeve. With additional improvements made in the design for the preventing blood seepage eliminates the possible blood contamination, this invention provides total safety working condition, and feasibility for assembly and production. This invention has avoided the many disadvantage of the current design, fulfilled the requirements for long time infusion, and repeated penetration. This design has reached the goal for easy operating, and at the same time minimizing vessel injury.

## Claims

1. A safety transfusion needle catheter comprises a flexible retention tube fitted over a metal guiding needle with a wedge-shaped needle tip. The flexible retention tube is provided with a needle handle connected with it, it is **characterized by**:
The angle between the end of the flexible retention tube and its longitudingnal axis or longitudingnal tube was is less than 90°;
The cross section of the needle handle has an outer contour line of a closed curve formed with a curve intersecting a beeline.

2. A safety transfusion needle catheter as claimed in claim 1, it is **characterized by**:
The cutting line of the end of the flexible retention tube is parallel to the opening of the metal guide needle;
There is a chamfer structure on the opening end of the wall of the flexible retention tube. The surface of the edge of the opening of the flexible retention tube is polished;
There is a round-smooth transition made in the connection between the edge of the opening and the out wall of the flexible retention tube.

3. A safety transfusion needle catheter as claimed in claim 1, it is **characterized by**:
The curve described is a circular arc curve, elliptical arc curve, droplet-type curve or their combinations.

4. A safety transfusion needle catheter as claimed in claim 1, it is **characterized by**:
The outer contour line of the cross section of the needle handle described above is semicircular, semielliptical, semi drop-shaped, approximate semicircular, approximate semielliptical, approximate semi drop-shaped, or their combinations.

5. A safety transfusion needle catheter as claimed in claim 1, it is **characterized by**:
The needle handle described above is a two pieces-hinge structure that can be buckled together. There are hasp buckles on the two pieces-hinge structure, respectively. On the inner face-to-face side of two pieces-hinge structure there is a furrow that allows flexible retention tube to be lay between two pieces-hinge structures.

6. A safety transfusion needle catheter as claimed in claim 1, it is **characterized by**:
On the surface of the needle handle there are embossing non-skid tread.

7. A further safety transfusion catheter comprises a flexible retention tube fitted over a metal guiding needle, a transfusion connecting tube in fluid communication with flexible retention tube and a rubber or silicon casing plug at the end of the flexible retention tube. The back end of the metal guiding needle is in telescoping engagement with a hollow sleeve. The transfusion connecting tube and flexible retention tube form a T-shaped or an oblique Y-shaped structure. This is **characterized by**:
A safety protective sheath shorter than the metal guiding needle is provided at the front end of the safety hollow sleeve cap.
A spring is provided between the end hub of the metal guiding needle and the front end of the hollow sleeve.
A seesaw typed stop block is provided in the hollow sleeve.
A reset-lock button is provided on the portion of the hollow sleeve having the seesaw typed stop block.

8. A safety transfusion needle catheter as claimed in claim 7, it is **characterized by**:
The metal guiding needle is slidably fitting in the hollow protection sleeve;
The spring is put around the metal guiding needle;
A seesaw type stop blocker is fitted at the end of the metal guiding needle that allows the needle can be completely out of the sleeve into to the flexible catheter tube;
An additional locking click board is fitted on the seesaw type stop block to keep the metal needle inserted through the rubber (or silicon) plug into the flexible catheter tube while it in the operating position even when the tip of the metal guiding needle be pulled out from the rubber (or silicon) plug manually. Sliding the locking click board to release the seesaw type stop block, the tip of the metal guiding needle can be completely retracted into the safety sleeve.

9. A safety transfusion needle catheter as claimed in claim 7, it is **characterized by**:
In the conjunction of the infusion tube connection and the flexible infusion tube described above adding a wing-shaped or a butterfly-shaped adjunct handle. One side of the handle is flat, on the surface of at lest one side of the handle has embossing non-skid tread.

10. A safety transfusion needle catheter as claimed in claim 7, it is **characterized by**:
The handle is a two pieces-hinge structure buckled together, it can be separately provided on the conjunction of the infusion tube connection and the flexible infusion tube.
